# DEMANDE DE BREVET EUROPEEN

(11) **EP 1 967 177 A2**
(43) Date de publication de la demande: **10.09.2008**
(21) Numéro de dépôt: 08101299.9
(22) Date de dépôt: 06.10.2004
(51) Int. Cl.: A61K 8/39, A61K 8/67, A61K 8/92, A61Q 17/02, A61Q 19/00, A61K 8/36, A61K 8/34, A61K 8/97, A61Q 13/00

(54) **Composition dermo-cosmétique pour animaux de compagnie**

(30) Priorité: 06.10.2003 FR 0311687
(62) Demande divisionnaire de: 04791473.4
(71) Demandeur: Laboratoire de Dermo-Cosmetique Animale, 81100 Castres (FR)
(72) Inventeur: Fabries, Lionel, 81100, Castres (FR)
(74) Mandataire: Sartorius, Jérome

(57) **Abrégé**

La présente invention concerne une composition dermo-cosmétique mettant en oeuvre la capacité des glandes sébacées à stocker des principes actifs et à les relarguer par le sébum, la composition étant destinée à être appliquée sur la peau d'un animal et étant caractérisée en ce qu'elle comprend au moins :
- un solvant organique vecteur de diffusion de principes actifs de ladite composition à la surface de ladite peau,
- au moins un complexe d'huiles essenfielles,
- des acides gras poly-insaturés, utilisés par voie locale chez les animaux, et
- un produit insectifuge choisi parmi les produits suivants :
- huile de graines de Neem,
- huile de Ricin (*Ricinus Communis*),
- diméthyl Phtalate,
- éthyl hexane diol,
- camphre naturel et synthétique,
- pyrethre et dérivés (*pyrethum album*), et
- essence d'ail (*allium Sativum*).

## Description

La présente invention concerne une composition Dermo-Cosmétique pour animaux de compagnie (y compris les nouveaux animaux de compagnie NAC).

Les produits de dermatologie vétérinaire à but préventif ou d'entretien de l'animal sont conçus généralement sous forme de crèmes, ou de lotions ou de shampooings.

Leur application à l'animal est toujours délicate et parfois désagréable pour celui-ci.

La tendance normale de l'utilisateur est souvent de surdoser la quantité de produit appliqué, c'est le cas fréquent des lotions ou des poudres, ce qui a pour effet de perturber l'animal.

Les poudres, shampooings ou lotions sont souvent à effet localisé, ce qui ne permet pas de couvrir par une seule application l'ensemble des besoins d'un traitement Dermo-Cosmétique :
- Soin de la peau et hydratation de celle-ci,
- Effet apaisant,
- Effet désodorisant,
- Effet assainissant et purifiant,
- Effet antioxydant et anti-radicalaire,
- Effet insectifuge.

La présente invention vise à obvier à ces inconvénients en réalisant une composition dont la mise en oeuvre est facilitée et qui puisse dans sa formulation la plus élaborée répondre à une pluralité des besoins essentiels d'un traitement Derme-Cosmétique de l'animal,

La composition selon l'invention permet après une application localisée la diffusion des principes actifs sur toute la surface grâce au solvant vecteur de diffusion puis l'invention met en oeuvre la capacité des glandes sébacées à stocker dans certaines conditions les principes actifs du traitement et à les relarguer progressivement parla production naturels de sébum.

A cet effet, la composition Dermo-Cosmétique selon l'invention mettant en oeuvre la capacité des glandes sébacées à stocker le ou les principe(s) actifs et à les relarguer et à les diffuser par le sébum se caractérise essentiellement en ce qu'elle comprend au moins :
❖ Un vecteur qui est un solvant
❖ Un complexe d'huiles essentielles choisis seules ou en combinaison entre elles en fonction des effets à obtenir.

Cette combinaison permet de proposer une composition prête à l'emploi, essentiellement d'un vecteur qui est un solvant et d'huiles essentielles qui, appliquée en un ou plusieurs points du pelage de l'animal, réalise un traitement sur l'intégralité de la surface cutanée de l'animal pendant plusieurs jours.
Des essais réalisés ont démontré l'obtention d'un résultat sur 7 jours au moins.

Suivant une autre caractéristique de invention, le solvant constituant Vecteur est un ethoxydiglycol.

Suivant une autre caractéristique, la composition comprend :
❖ Un vecteur constitué d'un solvant organique,
❖ Un complexe d'huiles essentielles,
❖ Des acides gras poly-insaturés, utilisés par voie locale chez les animaux.

Suivant encore une autre caractéristique de l'invention, la composition ci-dessus énoncée peut comporter un agent apaisant et/ou un agent anti-inflammatoire et/ou à effet anti-prurigineux.

D'autres- avantages et caractéristiques de l'invention apparaîtront à la lecture de la description ci après.

La composition selon l'invention vise à rester un traitement de la peau de l'animal qui ait plusieurs effets :
- Prévention et hydratation ;
- Effet apaisant ;
- Effet désodorisant:
- Effet assainissant et purifiant ;
- Effet antioxydant et anti-radicalaire ;
- Effet insectifuge.

L'intérêt de l'invention est de pouvoir conjuguer tout ou partie des effets ci-dessus, selon la complexité de la composition à partir d'une application sur une zone réduite de l'épiderme de l'animal.

L'invention est basée sur l'association d'une synergie aromatique unique et novatrice à d'autres actifs hydratants, apaisants, antioxydants et nourrissants de la peau.
Chacune des huiles essentielles entrant dans la composition fait l'objet d'une définition qualitative précise basée sur les critères EOBBD (Essential Oil Botanically and Biochemically defined, iso 9002 certified Quality Assurence Process).

Une seule application suffit pour un animal de petite taille ; deux seront nécessaires pour un animal de plus grande taille.

Suivant une formulation basique, la composition selon l'invention comprend au moins un vecteur qui est constitué d'un solvant organique ou d'un équivalent et un complexe concentré d'au moins deux huiles essentielles.

La présence du vecteur permet l'application de la composition en un ou deux points de la surface cutanée, la diffusion du produit, son stockage par les glandes sébacées et son relargage progressif.

A ce jour, la capacité du vecteur n'était utilisée que pour apporter à l'animal un produit insecticide,
L'intérêt de l'invention est de proposer un traitement complet de l'épiderme et du pelage qui ait un effet assainissant, apaisant et calmant sur l'animal:
- Par la restauration d'un écosystème cutané équilibré,
- L'invention permet d'éviter des désordres inflammatoires et infectieux de l'épiderme et l'effet apaisant et calmant de la composition apporte à l'animal une sensation de bien être et de fraîcheur qui est régulatrice de son humeur.

L'originalité de la composition permet de baigner l'animal 48 heures avant ou après le traitement, tout en bénéficiant de l'efficacité de la composition appliquée pendant une semaine au moins.

Le solvant organique constituant le vecteur peut être de préférence un solvant, de polarité intermédiaire par exemple :
➢ Un ethoxydiglycol ou un butyleneglycol, ou un propyléneglycol, ou un glycérine dans la proportion des 1 % à 99 % de la composition totale en fonction de ses autres constituants.

Plus généralement, le vecteur est choisi parmi le groupe :
Acétone, acétonitrile, alcool benzylique, butyldiglycol, diméthylacétamide, diméthyformamide, éther n-butylique du dipropylèneglycol, éthanol, isopropanol, méthanol, éthylèneglycol monoéthyléther, monométhylacétamide, monométhyléther de dipropylèneglycol, polyoxyéthylène-glycols liquide, propyléneglycol, 2-pyrrolidone, notamment N-méthyl pyrrolidone, monoéthylèther de diéthyléneglycol, éthylèneglycol, diéthylphtalate, et mélange d'au moins deux d'entre eux.

La composition ci-dessus peut-être complétée par des acides gras essentiels poly-insaturés pour l'amélioration de la surface cutanée.

Les acides gras poly-insaturés oméga 3 et oméga 6 peuvent être apportés par l'adjonction d'huile de graine de chanvre (cannabis sativa) dans une proportion qui peut être de 1% à 15% de la composition totale ou par des substituts naturels tels que :
- huile de poisson (orange Roughy),
- huile de Bourrache (Borago Officinalis),
- huile de coco (Cocos Nucifera),
- huile de Kukui,
- huile de sésame (Sesamum Indicum),
- AGPI de synthèse reconstitués ou extraits naturels,
- Acide Elcopentaenoïque (EPA) et
- Acide Docosahexaenoïque (DHA),
- Beurre de karité (Shea Butter),
- Huile d'olive (Olea Europea),
- Huile de colza,
- Huile de noix,
- Huile de Soja,
- Huile de Buglosse (echium Plantagineum),

Qui peuvent être ajoutés à la composition dans la proportion de 1 à 99 %, plus particulièrement de 2 % à 50 % et de préférence de 5 % à 10 % de la composition totale.

La composition peut également recevoir un agent apaisant et/ou anti-inflammatoire et/ou anti-prurigineux,

De préférence, l'agent apaisant est le Laureth 9 dans la proportion de 0,8 % à 3 % ou ses substituts tels que :
Menthol, et ses sels, procaïne, lidocaïne, corticoïdes, seuls ou en combinaison d'au moins deux d'entre eux.

Le Polymère Laureth 9 est un mélange d'éthers monolauriques de polyéthylène glycols ayant une moyenne de 9 groupes éthylènes-oxyde par molécule.

L'agent anti-inflammatoire peut-être le Bisabolol dans la proportion de 1% à 5 % ou ses substituts ;
- Azuléne naturel ou synthétique,
- allantoïne,
- acide 18 bêta glycyrrhetinique et ses sels
dans la proportion de 0,01 % à 10 % et préférentiellement de 0,01 à 1 %
- extraits d'aloès,
- extraits de calendula,
- extrait de carotte,
dans la proportion de 0.01 à 10 %.et préférentiellement de 5 à 10 %.

Le Bisabolol aussi dénommé l'Alpha-Bisabolol a la formulation suivante :
1-methyl-4 (1,5-dimethyl-1 hydroxyhex-4(5)-enyl)-cyclohexen-1 ; 6-méthyl-2-(4-methyl-3-cyclohexen-1 yl)-5-hepten-2ol.

La composition ci-dessus comprend un produit antioxydant et anti-radicalaire : le Tocophérol ou vitamine E, sous forme de Tocophéryl acétate dans la proportion de 0,2 % à 2 % ou ses substituts tels que Tocophéryl palmitate ou Tocophéryl linoleate.

La composition comprend également en combinaison avec l'un quelconque des composés ci-dessus un agent filmogène inhibiteur de cristallisation épaississant et stabilisant tel que le produit Polivinylpyrrolidone ou choisi parmi le groupe :
Hydroxyméthyl cellulose, alcools polyvinyliques, copolymères d'acétate de vinyle et de vinylpyrrolidone, polyéthyléneglycols, alcool benzylique, mannitol, glycérol, sorbitol, esters de sorbitane polyoxyéthylénés, lécithine, carboxyméthylcellulose sodique, dérivés acryliques tels que méthacrylates dérivés des polyesters du type des copolymères triméthylpentanedjol / acide adipique et autres.

La composition peut également comporter un produit insectifuge tel que de l'huile de graine de NEEM qui est en fait Azadirachta indica titré à 0,25% en Azadirachtine ou ses substituts tels que;
o huile de Ricin (Ricinus Communis),
o diethyltoluamide et butylacetylaminopropionate,
o dimethyl Phtalate,
o Ethyl hexane diol,
o Camphre naturel et Synthétique,
o Pyrèthre et dérivés (pyrethum album),
o Essence d'ail (allium Sativum).

Plus généralement, la composition selon l'invention peut comporter seule ou en combinaison les huiles essentielles suivantes ;
o Rosmarinus officinalis dans la proportion de 0,3% à 0,9% qui a un effet assainissant purifiant et apaisant ou de l'Eucalyptol,
o Lavandula hybrida dans la proportion de 0,3% à 0,9% ou lavandula angustifolia qui a un effet désodorisant.
o Eugenia caryophyllus qui a un effet répulsif pour les insectes, dans la proportion de 0,3% à 0,9%.
o Melaleuca Alternifolia ou origan majorana dans la proportion de 0,3% à 0,9% qui a un effet anti-infectieux.
o Cinnamorum Camphora ou Camphor, dans la proportion 0,15% à 0,6% à effet désodorisant et antiprurigineux.
o Mentha Piperita ou mentha orvensis dans la proportion de 0,15% à 0,6% à effet désodorisant.
o Cedrus atlantica ou SAS libanus, dans la proportion de 0,15% à 0,6% à effet anti-parasitaire, cicatrisant et anti-carchogène.
o Curcuma longa ou curcuma zedoaria dans la proportion de 0,15% à 0,6% qui a également un effet anti-parasitaire.
o Origanum compactum ou origonum vulgare, dans la proportion de 0.06% à 0,3% à effet de répulsifs des insectes et anti-microbien,
o Gaultheria procumbens ou son substitut méthylsalicylate à effet calmant et antiprurigineux.
o Musk synthétique ou musk ketone, dans la proportion de 0,06% à 0,3% pour parfumer ou masquer les odeurs naturelles.

Les composés ci-dessus sont utilisés en combinaison dans la composition selon l'invention en fonction des effets recherchés.

Plus généralement, les huiles essentielles de la liste jointe peuvent être combinées à la composition selon l'invention.

| **SUBSTITUTS COMPLEXE D'HUILES ESSENTIELLES** | | | | |
|---|---|---|---|---|
| Ajowan | Amandier amer | Ammi, khella | Aneth odorant | Angélique |
| Armose herbe blanche | Basmic | Bay | Bergamotier | Bois de rose |
| Bois de santal | Boldo | Bucchu | Cabreuva | Cajeput |
| Calamus | Camomille romaine ou Noble | Camphrier | Cannelier de Ceylan | Cannelle de Chine |
| Cardamones | Carotte | Carvi | Cataire | Cèdre de l'Atlas |
| Cèdre de Virginie | Céleri | Chénopode vermifuge | Ciste ladanifére | Citron jaune |
| Citronnelle de Ceylan | Citronnelle de Java | Copahu | Coriandre | Cubèbe |
| Cumin | Curcuma | Cyprès toujours vert | Douglas | Elémi |
| Encens | Epinette noire | Estragon | Eucalyptus | Galac |
| Galbanum | Gaulthérie, Wintergreen | Genévrier commun | Géranium odorant | Gingembre |
| Giroflier | Hélichryse. | Hysope à rameaux couchés | Hysope officinale | Inule |

| | | | |
|---|---|---|---|
| lorménie, Camomille sauvage | Lantane | Laurier noble | Lavande aspic |
| Lavande officinale | Lavande stoechade | Lavandin | Lemon-grass |
| Lentisque pistachier | Lepstosperme citronné. | Limette | Litsée |
| Livèche | Mandarine rouge | Marjolaine à coquilles | Marjolaine sylvestre |
| Marjolaine vivace | Matricaire | Mélèze | Mélisse véritable |
| Menthe des champs | Menthe poivrée | Menthe pouliot | Millefeuille |
| Millepertuis | Muscade | Myrrhe | Myrte |
| Nard | Niaouli | Oranger | Oranger doux |
| Origan | Origan d'Espagne | Origan vulgaire | Palma - Rosa |
| Pamplemousse | Patchouli | Persil | Pin des Landes |
| Pin Sylvestre | Poivre noir | Vinylpyrrolidone aromatique | Romarin officinal |
| Romarin pyramidal | Rose de Bulgarie | Santal amyris | Santoline petit cyprès |
| Sapin baumier | Sapin de Sibérie | Sapin pectiné | Sarriette vivace |
| Sassafras | Sauge à petite feuille | Sauge officinale | Sauge sclarée |
| Serpolet | Tanaisie bleue | Tea tree | Térébenthine |
| Thuya | Thym | Thym à feuilles de sarriette | Verge d'or |
| Vergerette | Verveine odorante | Vétiver | Ylang-ylang, distillation complète |
| Ylang-ylang, tête de cuvée | | | |

Les huiles essentielles sélectionnées l'ont été en fonction d'un cahier des charges précis quant aux principes actifs qu'elles contiennent. Parmi l'ensemble des actifs figurent en particulier des sesquiterpènes, aux propriétés anti-inflammatoires, des composés phénolés aux propriétés anti-infectieuses et immunostimulantes localement, d'autres composés phénolés (eugénol et carvacrol) aux effets insectifuges et répulsifs et des esters aux propriétés antalgiques et décongestionnantes susceptibles de tempérer voire d'empêcher une action éventuellement irritante des phénols au niveau cutané.

Une formule particulièrement adaptée de la composition selon l'invention comprend :
- A titre de vecteur et de solvant cutané un ethoxydigiycol.
- A titre d'huile végétale riche en acides gras poly-insaturés, de l'huile de chanvre (cannabis saliva).
- A titre d'huiles essentielles concentrées :
   ▩ Rosmarius officinalis, lavendula hybrida,
   ▩ Eugénia Caryopilyllus, Melaleuca Alternifolia
   ▩ Cinnamomum Camphora, Mentha Piperita
   ▩ Cedrus Atlantica, Curcuma Longa, Origanum Compactum,
   ▩ Gaultheria Procumbens, Musk
- A titre de produit insectifuge et apaisant, de l'extrait de graines de Neem (azadirachta Indica titrée à 0,25% en Azadirachtine).
- A titre d'actifs apaisant, désensibilisant et antiprurigineux, le Laureth 9.
- Un agent épaississant stabilisant du produit, anticristallisation et formant un film sur la peau de l'animal, Polivinylpyrrolidone tel qu'énoncé plus haut dans la description.
- Une vitamine E à fonction antioxydante, telle que D Alpha Tocophérol ou Tocophéryl acétate, ou les produits mentionnés plus haut dans la description, ou les produits mentionnés plus haut (autres antioxydants) : butylhydroxyanisole, butylhydroxytoluene, acide ascorbique, metabisulfate de sodium, gallate de propyle, thiosulfate de sodium, mélange d'au plus deux d'entre eux.
- Un agent anti-inflammatoire, tels que l'Alpha-Bisabolol, le Bisabolol ou ses substituts tels que mentionnés plus haut dans la description.

Les principes actifs de la formulation pourront être intégralement ou partiellement inclus dans une formulation "retard" permettant un allongement du temps de relargage et donc de la durée d'action de ces principes actifs. Les composés permettant cette modification pourront être choisis parmi les bêta-cyclodextrines, la silice, les fibres de cellulose, le nylon, les poly-méthacrylates.

Une formule retard ou à libération programmée peut permettre une libération prolongée des principes actifs jusqu'à vingt-huit jours après l'application. Les formules retard font usage de :
- Bêta cyclodextrines, sucres cycliques d'origine naturelle : présents dans la formulation en concentration variant de 2 à 10 %.
- Et/ou fibres de cellulose, originaires du bois : présents dans la formulation en concentration variant de 2 à 10 %.
- Et/ou sphères de polyméthyleméthacrylate et de nylon - présents dans la formulation en concentration variant de 4 à 12 %.
- Et/ou microréservoirs de silice, enrobés ou non enrobés : présents dans la formulation en concentration variant de 2 à 10 %.
- Et/ou polymères polyesters linéaires de masse moléculaire de 800 à 5000 occlusifs Daltons et retensifs : présents dans la formulation en concentration variant de 10 à 20 %.

Les proportions de chacun des composants sont prises dans les valeurs de pourcentages énoncés plus haut, ces valeurs étant exprimées en pourcentage de la composition totale et compléte.

## Revendications

1. Composition dermo-cosmétique mettant en oeuvre la capacité des glandes sébacées à stocker des principes actifs et à les relarguer par le sébum, la composition étant destinée à être appliquée sur la peau d'un animal et étant **caractérisée en ce qu'**elle comprend au moins :
- un solvant organique vecteur de diffusion de principes actifs de ladite composition à la surface de ladite peau,
- au moins un complexe d'huiles essentielles,
- des acides gras poly-insaturés, utilisés par voie locale chez les animaux, et
- un produit insectifuge choisi parmi les produits suivants
- huile de graines de Neem,
- huile de Ricin *(Ricinus Communis),*
- diméthyl Phtalate,
- éthyl hexane diol,
- camphre naturel et synthétique,
- pyrethre et dérivés *(pyrethum album),* et
- essence d'ail *(allium Sativum).*

2. Composition selon la revendication 1, **caractérisée en ce que** le solvant vecteur est un éthoxydiglycol.

3. Composition selon la revendication 1, **caractérisée en ce que** le solvant vecteur est choisi dans le groupe suivant :
acétone, acétonitrile, alcool benzylique, butyldiglycol, diméthylacétamide, diméthyformamide, éther n-butylique du dipropylèneglycol, éthanol, isopropanol, méthanol, éthylèneglycol monoéthyléther, monométhylacétamide, monométhyléther de dipropylèneglycol, polyoxyéthylène-glycols liquide, propylénegiycol, 2-pyrrolidone, monoéthyléther de diéthylèneglycol, éthylèneglycol, diéthylphtalate, et mélange d'au moins deux d'entre eux.

4. Composition selon l'une quelconque des revendications précédentes, **caractérisée en ce que** les acides gras sont choisis dans les huiles de chanvre, dans une proportion de 1 à 15%.

5. Composition selon l'une quelconque des revendications 1 à 3, **caractérisée en ce que** les acides gras sont apportés par des produits choisis dans le groupe :
- huile de poisson (orange Roughy)
- huile de bourrache *(Borago Officinalis)*
- huile de coco (Cocos *Nucifera)*
- huile de Kukui
- huile de sésame *(Sesamum Indicum)*
- AGPI de synthèse reconstitués ou extraits naturels
- acide eicopentaenoïque (EPA)
- acide docosahexaenoïque (DHA),
- beurre de karité (Shea Butter)
- huile d'olive *(Olea Europea)*
- huile de colza
- huile de noix
- huile de soja
- huile de buglosse *(echium Plantagineum)*
dans une proportion de 3 à 50 %.

6. Composition selon la revendication précédente, dans laquelle la proportion desdits acides gras est comprise entre 5 et 10 %.

7. Composition selon l'une quelconque des revendications précédentes, **caractérisée en ce qu'**elle comprend un agent apaisant et/ou anti-inflammatoire et/ou à effet anti-prurigineux,

8. Composition selon la revendication 1, constituée :
- d'un solvant,
- d'un complexe d'au moins deux huiles essentielles,
- d'acides gras poly-insaturés oméga 3 et oméga 6,
- d'un agent apaisant et/ou anti-inflammatoire et/ou à effet anti-prurigineux,
l'agent apaisant étant le laureth 9, ou ses substituts, seuls ou en combinaison d'au moins deux d'entre eux.

9. Composition selon la revendication précédente, lesdits substituts étant choisis parmi la procaïne, la lidocaïne, les corticoïdes, le menthol.

10. Composition selon la revendication 8, **caractérisée en ce que** l'agent anti-inflammatoire est le bisabolol ou l'un au moins de ses substituts.

11. Composition selon la revendication précédente, ledit au moins un des substituts du bisabolol étant choisi dans le groupe :
- azuléne naturel ou synthétique,
- allantoïne,
- acide 18 bêta glycyrrhetinique et ses sels,
- extraits d'aloès,
- extraits de *calendula* ou de carotte
dans la proportion de 0,01 à 10 %.

12. Composition, selon l'une quelconque des revendications précédentes, **caractérisée en ce qu'**elle comprend un agent filmogène inhibiteur de cristallisation, épaississant et stabilisant.

13. Composition selon la revendication précédente, dans laquelle l'agent filmogène est une polyvinylpyrrolidane ou est choisi parmi le groupe ;
hydroxyméthyl cellulose, alcools polyvinyliques, copolymères d'acétate de vinyle et de vinylpyrrolidone, polyéthylèneglycols, alcool benzylique, mannitol, glycérol, sorbitol, esters de sorbitane polyoxyéthylénés, lécithine, carboxymèthylcellulose sodique, dérivés acryliques tels que méthacrylates et autres,

14. Composition selon l'une quelconque des revendications précédentes, **caractérisée en ce qu'**elle comprend :
- à titre de vecteur et de solvant cutané, un éthoxydiglycol,
- à titre d'huile végétale riche en acides gras poly-insaturés, de l'huile de chanvre,
- à titre d'huiles essentielles concentrées :
- *Rosmarius officinalis, lavendula hybrida,*
- *Eugenia Caryophyllus, Melaleuca Alternifolia,*
- *Cinnamomum Camphora, Mentha Piperita,*
- *Cedrus Atlantica, Curcuma Longa, Origanu*m *Compactum,*
- *Gaultheria Procumbens,* Musk,
- à titre de produit insectifuge, de l'extrait de graines de Neem (azadirachta *Indica* titrée à 0,25 % en Azadirachtine),
- à titre d'actif apaisant, désensibilisant et antiprurigineux, le laureth 9,
- un agent épaississant stabilisant du produit, anticristallisation et formant un film sur la peau de l'animal,
- une vitamine E à fonction antioxydante,
- un agent anti-inflammatoire.

15. Composition selon l'une quelconque des revendications précédentes, **caractérisée en ce qu'**elle comprend une formule retard permettant une libération prolongée des principes actifs.

16. Composition selon la revendication précédente, **caractérisée en ce que** ladite formule retard fait usage de :
- silice et/ou
- bêta cyclodextrines, sucres cycliques d'origine naturelle présents dans la formulation en concentration variant de 2 à 10%, et/ou
- fibres de cellulose, originaires du bois : présents dans la formulation en concentration variant de 2 à 10 %, et/ou
- sphères de polyméthyleméthacrylate et de nylon: présents dans la formulation en concentration variant de 4 à 12 %, et/ou
- microréservoirs de silice, enrobés ou non enrobés : présents dans la formulation en concentration variant de 2 à 10 %, et/ou
- polymères polyesters linéaires de masse moléculaire de 800 à 5000 Daltons occlusifs et rétensifs : présents dans la formulation en concentration variant de 10 à 20 %.

17. Composition selon l'une quelconque des revendications précédentes, déterminée de manière que, appliquée en un ou plusieurs points d'un pelage de l'animal, elle réalise un traitement sur l'intégralité de la surface cutanée de l'animal pendant plusieurs jours.

18. Procédé de traitement non thérapeutique comportant les étapes suivantes :
a) application, en un ou deux points de la surface cutanée d'un animal à pelage, d'une composition cosmétique conforme à l'une quelconque des revendications 1 à 13 et 15 à 17,
b) diffusion de ladite composition,
c) stockage de ladite composition par des glandes sébacées et relargage progressif,

19. Procédé selon la revendication précédente, dans lequel on réalise un traitement sur l'intégralité de la surface cutanée de l'animal pendant plusieurs jours.

20. Procédé selon l'une quelconque des revendications 18 à 19, dans lequel l'animal est un animal de compagnie.
